# EUROPEAN PATENT APPLICATION

(11) **EP 4 832 012 A1**
(43) Date of publication of application: **28.10.2026**
(21) Application number: 26171397.8
(22) Date of filing: 09.04.2026
(51) Int. Cl.: G16H 50/50, A61B 34/10, G16H 50/20

(54) **TECHNIQUES FOR PREDICTING A PRE-MORBID STATE OF A MORBID BONE**

(30) Priority: 15.04.2025 US 202563788845 P
(71) Applicant: Mako Surgical Corp., Weston, FL 33331 (US)
(72) Inventor: de Souza, Kevin Michael Augustine, Ilkley, LS29 7PB (GB); Robertson, Seonaid, Dunfermline, KY11 8PS (GB)
(74) Representative: Schott, Jakob Valentin

(57) **Abstract**

Systems, methods, software, and techniques for predicting a pre-morbid state of a morbid bone are provided. A pre-morbid state of a morbid bone model may be generated to estimate the pre-morbid state of the morbid bone. Generation of the pre-morbid state of the morbid bone model includes receiving a morbid bone model including a morbid portion, obtaining a mask, applying the mask to the morbid bone model to generate a masked morbid bone model, obtaining a statistical shape model defined based on healthy bone models, and fitting the statistical shape model to the masked morbid bone model.

## Description

### CROSS-REFERENCE TO RELATED APPLICATIONS

The subject application claims priority to and all the benefits of United States Provisional Patent App. No. 63/788,845 filed April 15, 2025, the entire contents of which are hereby incorporated by reference.

### BACKGROUND

Conventionally, planning a surgical or clinical procedure for treating patient anatomy involves generating a surgical plan based on a model of the patient anatomy derived from medical imaging data. Such models typically represent the anatomy in its current, observed state. However, in certain clinical scenarios, the current state of the patient anatomy may not accurately reflect an underlying or pre-morbid anatomical condition that is relevant for planning treatment.

For example, when planning treatment involving a morbid bone, conventional techniques generate a surgical plan directly from a model of the morbid bone. A morbid bone may include pathological features such as osteophytes, deformities, or erosion resulting from disease progression or degeneration. Because these morbid features are present in the anatomical model used for planning, they may influence or distort anatomical landmarks, surface geometry, or overall bone shape relied upon during surgical planning. As a result, surgical plans generated from such morbid bone models may be less representative of the patient's pre-morbid anatomy and may reduce the accuracy or consistency of the resulting surgical plan.

### SUMMARY

This Summary introduces a selection of concepts in a simplified form that are further described below in the Detailed Description below. This Summary is not intended to limit the scope of the claimed subject matter and does not necessarily identify each and every key or essential feature of the claimed subject matter.

In a first aspect, a computer program product comprises instructions executable by one or more processors to: receive a morbid bone model; obtain a mask defined based on a bone region statistically identified as being osteophyte prone; apply the mask to the morbid bone model to generate a masked morbid bone model; obtain a statistical shape model defined based on healthy bone models; and fit the statistical shape model to the masked morbid bone model to generate a pre-morbid bone model representing an estimated pre-morbid state of the morbid bone model.

In a second aspect, a computer program product comprises instructions executable by one or more processors to: receive a morbid bone model; obtain a mask defined based on a bone region statistically identified as being osteophyte-prone; apply the mask to the morbid bone model to remove portions of the morbid bone model and to generate a masked morbid bone model; obtain a statistical shape model defined based on healthy bone models; and fit the statistical shape model to the masked morbid bone model to generate a pre-morbid bone model representing an estimated pre-morbid state of the morbid bone model.

In a third aspect, a computer program product comprises instructions executable by one or more processors to: receive a bone model including a mesh formed of a plurality of triangles having vertices; obtain a mask defined based on vertices of the bone model to be disregarded, wherein the mask includes a plurality of weighting values corresponding to the vertices of the bone model to be disregarded; and apply the mask to the bone model to generate a modified bone model by assigning the weighting values to the corresponding vertices of the bone model, wherein the modified bone model includes the mesh of the bone model modified by the assigned weighting values.

In a fourth aspect, a computer program product comprises instructions executable by one or more processors to: receive anatomical model data representing a bone in a current anatomical state; identify one or more regions of the anatomical model data associated with pathological or non-representative anatomical features based on population-level data; modulate an influence of the one or more identified regions during anatomical modeling; and generate an estimated anatomical model representing a non-pathological or pre-pathological state of the bone based at least in part on anatomical information from regions outside the identified regions.

Also provided are surgical systems including a computing device or controller that is configured to implement the features of any preceding aspect, and a computer-implemented method of implementing the steps of any preceding aspect.

Each of the above aspects may be combined in-part, or in-whole, with any of the below implementations.

In some implementations, the morbid bone model is a bone model of a distal femur. In some implementations, the mask is defined based on a condylar region of the distal femur statistically identified as being osteophyte prone. In some implementations, the mask is further defined as outlining a periphery of a medial condyle and a lateral condyle of the distal femur.

In some implementations, the morbid bone model is a bone model of a proximal tibia. In some implementations, the mask is defined based on a condylar region of the proximal tibia statistically identified as being osteophyte prone. In some implementations, the mask is further defined as outlining a periphery of a lateral condyle of the proximal tibia and outlining a periphery of a medial condyle of the proximal tibia.

In some implementations, the statistical shape model is further defined as being trained on healthy bone models.

In some implementations, the pre-morbid bone model is formed of the fitted statistical shape model. In some implementations, the pre-morbid bone model is formed in-part by the fitted statistical shape model and formed in-part by the masked morbid bone model.

In some implementations, the morbid bone model includes a mesh formed of a plurality of triangles having vertices; the mask includes a plurality of weighting values corresponding to at least some vertices of the morbid bone model; and the instructions, when executed by one or more processors, are configured to apply the mask by being configured to assign the weighting values to the corresponding vertices of the morbid bone model, and wherein the masked morbid bone model includes the mesh of the morbid bone model modified by the assigned weighting values. In some implementations, a size and shape of the statistical shape model may be adjusted based on a size and shape of the masked morbid bone model to fit the statistical shape model to the masked morbid bone model.

In some implementations, the statistical shape model includes a mesh formed of a plurality of triangles having vertices; each of the plurality of vertices of the masked morbid bone model corresponds to a vertex of the statistical shape model; and the instructions, when executed by one or more processors, are configured to adjust a position of the plurality of vertices of the statistical shape model based on the weighting values of the plurality of vertices of the masked morbid bone model to adjust a size and shape of the statistical shape model.

In some implementations, the masked morbid bone model includes unremoved portions of the morbid bone model and does not include the removed portions of the morbid bone model, and wherein the fitted statistical shape model replaces the removed portions of the morbid bone model to generate the pre-morbid bone model.
In some implementations, portions of the morbid bone model within a geometry of the mask are removed, while preserving portions of the morbid bone model outside the geometry of the mask. In some implementations, the mask comprises a closed-off shape and wherein the instructions, when executed by one or more processors, are further configured to: apply the mask to the morbid bone model to create: a bounded portion of the morbid bone model bounded by the closed-off shape of the mask, and an unbounded portion of the morbid bone model adjacent to the closed-off shape of the mask; and preserve the bounded and unbounded portions of the morbid bone model to generate the masked morbid bone model. In some implementations, the mask comprises a first portion configured to outline a periphery of a lateral condyle of the proximal tibia, and a second portion configured to outline a periphery of a medial condyle of the proximal tibia. In some implementations, the first portion and the second portion of the mask each comprise an open shape and wherein the instructions, when executed by one or more processors, are further configured to: apply the mask to the morbid bone model to create a continuous portion of the morbid bone model and preserve the continuous portion of the morbid bone model to generate the masked morbid bone model.

In some implementations, the pre-morbid bone model may be utilized to generate a surgical or treatment plan for the morbid bone model. In some implementations, the pre-morbid bone model may be utilized to generate a diagnosis for the morbid bone model.

In some implementations, statistically identifying the osteophyte-prone bone region comprises analyzing aggregated clinical data across a population of subjects to determine bone regions exhibiting a higher likelihood, frequency, or magnitude of osteophyte formation relative to other bone regions. In some implementations, the aggregated clinical data may include imaging data, annotated anatomical models, surgical records, diagnostic outcomes, or combinations thereof. In some implementations, the statistical identification of osteophyte-prone regions does not require a particular statistical method, threshold, confidence interval, or machine learning technique, and may include any population-based determination indicating relative osteophyte prevalence or spatial consistency within the bone.

In some implementations, the mask may be represented as one or more of a surface geometry, a volumetric region, a vertex-level attribute map, a weighting field, or a combination thereof, and may be generated in a coordinate system corresponding to the morbid bone model or transformed into that coordinate system. In some implementations, applying the mask to the morbid bone model includes registering the mask to the morbid bone model by establishing correspondence between anatomical landmarks, mesh vertices, surfaces, or volumes of the mask and corresponding features of the morbid bone model. In some implementations, fitting the statistical shape model to the masked morbid bone model comprises optimizing one or more shape parameters of the statistical shape model to reduce a difference between the statistical shape model and preserved portions of the masked morbid bone model, wherein contributions from masked or removed portions are excluded or reduced. In some implementations, replacing removed portions of the morbid bone model with the fitted statistical shape model includes blending boundary regions between the preserved portions of the morbid bone model and corresponding portions of the fitted statistical shape model to maintain geometric continuity. In some implementations, fitting the statistical shape model to the masked morbid bone model is performed iteratively, wherein the mask, the masked morbid bone model, or both are updated between fitting iterations based on intermediate fitting results. In some implementations, the mask is generated by comparing the morbid bone model to a statistical shape model to identify one or more regions of the morbid bone model that deviate from the statistical shape model. In some implementations, regions exhibiting deviations exceeding a threshold are identified as osteophyte prone regions and are used to define the mask. In some implementations, the generated pre morbid bone model includes one or more confidence values associated with at least a portion of the bone model, the confidence values indicating a reliability of the estimated pre morbid state for corresponding regions of the bone. In some implementations, the statistical shape model is selected from a plurality of statistical shape models based on an anatomical similarity between the morbid bone model and a population of healthy bone models used to define each statistical shape model. In some implementations, the healthy bone models used to define the statistical shape model are selected based on one or more anatomical characteristics of the morbid bone model. In some implementations, the mask is generated dynamically for a specific morbid bone model during execution of the instructions.

In some implementations, the morbid bone model is generated from one or more medical imaging modalities including computed tomography (CT), magnetic resonance imaging (MRI), X-ray imaging, or combinations thereof. In some implementations, generating the surgical or treatment plan based on the pre-morbid bone model includes determining implant positioning, sizing, alignment, or resection parameters derived from the estimated pre-morbid anatomy. In some implementations, the fitting of the statistical shape model accommodates incomplete, noisy, or irregular portions of the morbid bone model by relying on preserved anatomical regions outside the osteophyte-prone region to guide estimation of the pre-morbid bone model.

### DESCRIPTION OF THE DRAWINGS

Advantages of the present invention will be readily appreciated as the same becomes better understood by reference to the following detailed description when considered in connection with the accompanying drawings wherein:
Figure 1 is a perspective view of a robotic surgical system for manipulating a target tissue of a patient with a tool, according to one example.
Figure 2 is a block diagram of a controller for controlling the robotic surgical system, according to one example.
Figure 3 is flowchart of a computer-implemented method of identifying portions of a bone model to be disregarded during surgical planning.
Figure 4 is a flowchart of a computer-implemented method of predicting a pre-morbid state of a morbid bone.
Figure 5 is a sample illustration of a step of receiving a morbid femur bone model of a morbid femur bone of the method of Figure 4.
Figures 6A-6C are rotated perspective views of an example mask defined based on a morbid portion of the morbid femur bone model of Figure 5.
Figure 7 is a sample illustration of a step of applying the mask to a masked morbid femur bone model of the method of Figure 4.
Figure 8 is a perspective view of a pre-morbid bone model, illustrating a pre-morbid state of the morbid femur bone model of Figure 5.
Figure 9 is a sample illustration of a step of receiving a morbid tibia bone model of a morbid tibia bone of the method of Figure 4.
Figures 10A-10C are rotated perspective views of an example mask defined based on a morbid portion of the morbid tibia bone model of Figure 9.
Figure 11 is a sample illustration of a step of applying the mask to a masked morbid tibia bone model of the method of Figure 4.
Figure 12 is a perspective view of a pre-morbid bone model, illustrating a pre-morbid state of the morbid tibia bone model of Figure 9.

### DETAILED DESCRIPTION

### I. Overview of the Robotic Surgical System

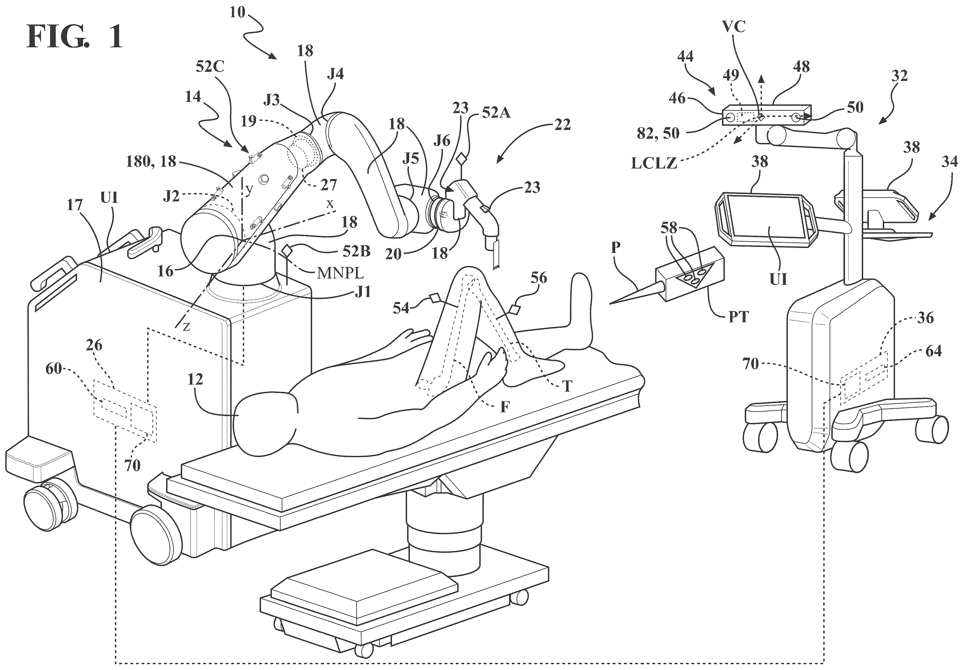

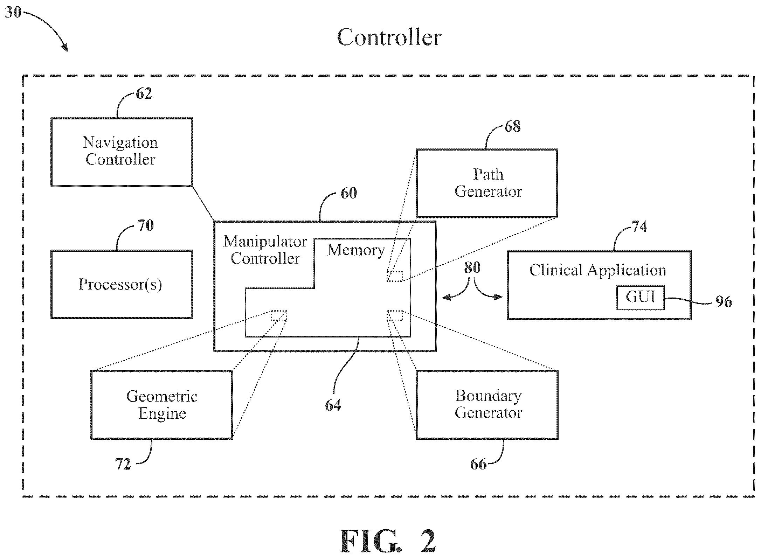

Referring to the Figures 1 and 2, wherein like numerals indicate like or corresponding parts throughout the several views, a surgical system 10 (hereinafter "system") and components thereof are illustrated. Further provided is a non-transitory computer readable medium comprising instruction, which when executed by one or more processors implement a software program or computer program product, as well as methods for using the software program or computer program product. Aspects of the software program and method, and details surrounding the various algorithms involved with each computer implemented step are addressed in the subsequent section below.

As shown in FIG. 1, the system 10 is a robotic surgical system for treating surgical site or anatomical volume (A) of a patient 12, such as bone or soft tissue. In FIG. 1, the patient 12 is undergoing a surgical procedure. The anatomy in FIG. 1 includes a femur (F) and a tibia (T) of the patient 12. The surgical procedure may involve tissue removal or treatment. Treatment may include cutting, coagulating, lesioning the tissue, treatment in place of tissue, or the like. In some examples, the surgical procedure involves partial or total knee or hip replacement surgery. In one example, the system 10 is designed to cut away material to be replaced by surgical implants, such as hip and knee implants, including unicompartmental, bicompartmental, multicompartmental, or total knee implants. Some of these types of implants are shown in U.S. Patent No. 8,250,028, entitled, "Prosthetic Implant and Method of Implantation," the disclosure of which is hereby incorporated by reference. The system 10 and techniques disclosed herein may be used to perform other procedures, surgical or non-surgical, or may be used in industrial applications or other applications where robotic systems are utilized.

The system 10 includes a manipulator 14. The manipulator 14 has a base 16 and plurality of links 18. A manipulator cart 17 supports the manipulator 14 such that the manipulator 14 is fixed to the manipulator cart 17. The links 18 collectively form one or more arms of the manipulator 14. The manipulator 14 may have a serial arm configuration (as shown in FIG. 1), a parallel arm configuration, or any other suitable manipulator configuration. In other examples, more than one manipulator 14 may be utilized in a multiple arm configuration.

In the example shown in FIG. 1, the manipulator 14 comprises a plurality of joints (J) and a plurality of joint encoders 19 located at the joints (J) for determining position data of the joints (J). For simplicity, only one joint encoder 19 is illustrated in FIG. 1, although other joint encoders 19 may be similarly illustrated. The manipulator 14 according to one example, has six joints (J1-J6) implementing at least six-degrees of freedom (DOF) for the manipulator 14. However, the manipulator 14 may have any number of degrees of freedom and may have any suitable number of joints (J) and redundant joints (J).

The manipulator 14 need not require "joint" encoders 19 but may alternatively or additionally utilize motor encoders. Also, the manipulator 14 need not require rotary joints, but may alternatively or additionally utilize one or more prismatic joints.

The base 16 of the manipulator 14 is generally a portion of the manipulator 14 that is stationary during usage thereby providing a fixed reference coordinate system (i.e., a virtual zero pose) for other components of the manipulator 14 or the system 10 in general. Generally, the origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or cart 17. The manipulator 14 and/or manipulator cart 17 house a manipulator computer 26, or other type of control unit. In other examples, the manipulator 14 can be a hand-held where the base 16 is a portion of the tool 20 held stable while the tool tip follows the path.

A surgical tool 20 (hereinafter "tool") couples to the manipulator 14 and is movable relative to the base 16 to interact with the anatomy in certain modes. The tool 20 is or forms part of an end effector 22 in certain embodiments. The tool 20 may be grasped by the operator. One exemplary arrangement of the manipulator 14 and the tool 20 is described in U.S. Patent No. 9,119,655, entitled, "Surgical Manipulator Capable of Controlling a Surgical Tool in Multiple Modes," the disclosure of which is hereby incorporated by reference. The manipulator 14 and the tool 20 may be arranged in alternative configurations. The tool 20 can be like that shown in U.S. Patent No. 9,566,121, entitled, "End Effector of a Surgical Robotic Manipulator," hereby incorporated by reference.

The tool 20 includes an energy applicator 24 designed to contact and remove the tissue of the patient 12 at the surgical site. In one example, the energy applicator 24 is a burr 25. The burr 25 may be substantially spherical and comprise a spherical center, radius (r), and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc.

Referring to FIG. 2, the system 10 includes a controller 30. The controller 30 may include software and/or hardware for controlling the manipulator 14. The controller 30 directs the motion of the manipulator 14 and controls a state (position and/or orientation) of the tool 20 with respect to a coordinate system. The controller 30 can control (linear or angular) velocity, acceleration, or other derivatives of motion of the tool 20. In one example, the coordinate system is the manipulator coordinate system MNPL, as shown in FIG. 1. One example of the manipulator coordinate system MNPL is described in U.S. Patent No. 9,119,655, entitled, "Surgical Manipulator Capable of Controlling a Surgical Tool in Multiple Modes," the disclosure of which is hereby incorporated by reference. Further aspects and capabilities of the controller 30 are described below.

The system 10 further includes a navigation system 32. One example of the navigation system 32 is described in U.S. Patent No. 9,008,757, filed on September 24, 2013, entitled, "Navigation System Including Optical and Non-Optical Sensors," hereby incorporated by reference. The navigation system 32 is configured to track movement of various objects. Such objects include, for example, the manipulator 14, the tool 20 and the anatomy, e.g., femur F and tibia T. The navigation system 32 tracks these objects to gather state information of each object with respect to a (navigation) localizer coordinate system LCLZ. Coordinates in the localizer coordinate system LCLZ may be transformed to the manipulator coordinate system MNPL, and/or vice-versa, using transformations.

The navigation system 32 includes a cart assembly 34 that houses a navigation computer 36, and/or other types of control units. A navigation interface is in operative communication with the navigation computer 36. The navigation interface includes one or more displays 38. The navigation system 32 is capable of displaying a graphical representation of the relative states of the objects tracked to the operator using the one or more displays 38. One or more input devices 40, 42 may be used to input information into the navigation computer 36 or otherwise to select/control certain aspects of the navigation computer 36. As shown in FIG. 1, such input devices 40, 42 include interactive touchscreen displays. However, the input devices 40, 42 may include any one or more of a keyboard, a mouse, a microphone (voice-activation), gesture control devices, and the like. The controller 30 may be implemented on any suitable device or devices in the system 10, including, but not limited to, the manipulator computer 26, the navigation computer 36, and any combination thereof.

The navigation system 32 also includes a navigation localizer 44 (hereinafter "localizer") coupled to the navigation computer 36. In one example, the localizer 44 is an optical localizer and includes a camera unit 46. The camera unit 46 has an outer casing 48 that houses one or more optical sensors 50.

The navigation system 32 includes one or more trackers. In one example, the trackers include a pointer tracker PT, one or more manipulator trackers 52, a first patient tracker 54, and a second patient tracker 56. In the illustrated example of FIG. 1, the manipulator tracker 52 is firmly attached to the tool 20 (i.e., tracker 52A), the first patient tracker 54 is firmly affixed to the femur F of the patient 12, and the second patient tracker 56 is firmly affixed to the tibia T of the patient 12. In this example, the patient trackers 54, 56 are firmly affixed to sections of bone. The pointer tracker PT is firmly affixed to a pointer P used for registering the anatomy to the localizer coordinate system LCLZ. The manipulator tracker 52 may be affixed to any suitable component of the manipulator 14, in addition to, or other than the tool 20, such as the base 16 (i.e., tracker 52B), or any one or more links 18 of the manipulator 14. The trackers 52, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the item that it is associated with.

Any one or more of the trackers may include active markers 58. The active markers 58 may include light emitting diodes (LEDs). Alternatively, the trackers 52, 54, 56 may have passive markers, such as reflectors, which reflect light emitted from the camera unit 46. Other suitable markers not specifically described herein may be utilized.

The localizer 44 tracks the trackers 52, 54, 56 to determine a state of each of the trackers 52, 54, 56, which correspond respectively to the state of the object respectively attached thereto. The localizer 44 provides the state of the trackers 52, 54, 56 to the navigation computer 36. In one example, the navigation computer 36 determines and communicates the state the trackers 52, 54, 56 to the manipulator computer 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

Although one example of the navigation system 32 is shown in the Figures, the navigation system 32 may have any other suitable configuration for tracking the manipulator 14 and the patient 12. In another example, the navigation system 32 and/or localizer 44 are ultrasound-based. For example, the navigation system 32 may comprise an ultrasound imaging device coupled to the navigation computer 36. The ultrasound imaging device images any of the aforementioned objects, e.g., the manipulator 14 and the patient 12, and generates state signals to the controller 30 based on the ultrasound images. The ultrasound images may be 2-D, 3-D, or a combination of both. The navigation computer 36 may process the images in near real-time to determine states of the objects. The ultrasound imaging device may have any suitable configuration and may be different than the camera unit 46 as shown in FIG. 1.

In another example, the navigation system 32 and/or localizer 44 are radio frequency (RF)-based. For example, the navigation system 32 may comprise an RF transceiver coupled to the navigation computer 36. The manipulator 14 and the patient 12 may comprise RF emitters or transponders attached thereto. The RF emitters or transponders may be passive or actively energized. The RF transceiver transmits an RF tracking signal and generates state signals to the controller 30 based on RF signals received from the RF emitters. The navigation computer 36 and/or the controller 30 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52, 54, 56 as shown in FIG. 1.

In yet another example, the navigation system 32 and/or localizer 44 are electromagnetically based. For example, the navigation system 32 may comprise an EM transceiver coupled to the navigation computer 36. The manipulator 14 and the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the controller 30 based upon EM signals received from the trackers. The navigation computer 36 and/or the controller 30 may analyze the received EM signals to associate relative states thereto. Again, such navigation system 32 examples may have structural configurations that are different than the navigation system 32 configuration as shown throughout the Figures.

The navigation system 32 and/or localizer 44 may have any other suitable components or structure not specifically recited herein. Furthermore, any of the techniques, methods, and/or components described above with respect to the camera-based navigation system 32 shown throughout the Figures may be implemented or provided for any of the other examples of the navigation system 32 described herein. For example, the navigation system 32 may utilize solely inertial tracking or any combination of tracking techniques.

As shown in FIG. 2, the controller 30 further includes one or more software programs and software modules. The software modules may be part of the program or programs that operate on the manipulator computer 26, navigation computer 36, or a combination thereof, to process data to assist with control of the system 10. The software programs and/or modules includes computer readable instructions stored in non-transitory memory 64 on the manipulator computer 26, navigation computer 36, or a combination thereof, to be executed by one or more processors 70a, 70b of the computers 26, 36. The memory 64 may be any suitable configuration of memory, such as RAM, non-volatile memory, etc., and may be implemented locally or from a remote database. Additionally, software modules for prompting and/or communicating with the operator may form part of the program or programs and may include instructions stored in memory 64 on the manipulator computer 26, navigation computer 36, or a combination thereof. The operator may interact with any of the input devices 40, 42 and the displays 38 to communicate with the software modules. The user interface software may run on a separate device from the manipulator computer 26 and navigation computer 36.

The controller 30 includes a manipulator controller 60 for processing data to direct motion of the manipulator 14. In one example, as shown in FIG. 1, the manipulator controller is implemented on the manipulator computer 26. The controller 30 further includes a navigation (or localization) controller 62 for communicating the state data relating to the femur F, tibia T, and manipulator 14 to the manipulator controller 60. The navigation controller 62 is also configured to process/analyze the measurements from the camera and trackers to compute the state data. This processing could be done elsewhere on the system, including inside the camera itself. The manipulator controller 60 receives and processes the state data provided by the navigation controller 62 to direct movement of the manipulator 14. As shown in FIG. 1, the navigation controller 62 is implemented on the navigation computer 36. The controllers 60 and 62 handle the real-time control and algorithms.

As shown in FIG. 2, the controller 30 includes a boundary generator 66. The boundary generator 66 is a software program or module that generates a planar or non-planar virtual boundary 70 for constraining movement and/or operation of the tool 20. Such virtual boundaries 70 may also be referred to as virtual meshes, virtual constraints, or the like. The virtual boundaries 70 may be defined with respect to an anatomical model, such as a 3-D bone model. The anatomical model (AM) is registered to the one or more patient trackers 54, 56 such that the virtual boundaries 70 become associated with to the anatomical model (AM). The manipulator controller 60 executes the virtual boundaries 70 by tracking the state of the tool 20 relative to the virtual boundaries 70. In one example, the state of the TCP is measured relative to the virtual boundaries 70 for purposes of determining haptic forces that are applied to a virtual model via a physics simulation. The results of the simulation are commanded to the manipulator 14. The controller 30 controls/positions the manipulator 14 in a manner that emulates the way a physical handpiece would respond to the user and virtual haptic forces. The boundary generator 66 may be implemented on the manipulator controller 60. Alternatively, the boundary generator 66 may be implemented on other components, such as the navigation controller 62.

The virtual boundaries 70 can be either implant-specific or patient-specific. Thus, it is not necessary to have patient-specific virtual boundaries 70 even for the case of patient-specific tool paths. For implant-specific scenarios, the boundary generator 66 does not need to be part of the system 10 or controller 30, but rather the boundaries 70 can be manually generated offline for each possible implant size, and the resulting boundaries 70 stored with the software. For patient-specific scenarios, the boundary generator 66 may be part of a clinical application 74 (described below) that handles user interaction, rather than part of the controller 30 or manipulator controller 60.

A milling path generator 68 is another software program or module run by the controller 30. In one example, the milling path generator 68 is run by the manipulator controller 60. The milling path generator 68 generates a milling path for the tool 20 to traverse, such as for removing sections of the anatomy to receive an implant.

A geometric engine 72 is another software program or module run by the controller 30. The geometric engine 72 may be a sub-set of the milling path generator 68 or may be a program or module separate from the milling path generator 68. The geometric engine 72 is configured to assist in milling path generation as well as providing on-the-fly flexibility for accommodating intraoperative changes to the surgical plan, e.g., implant placement.

The boundary generator 66, milling path generator 68, and geometric engine 72 may be sub-sets of a software program 80. Alternatively, each may be software programs that operate separately and/or independently in any combination thereof. The term "software program" is used herein to describe the computer-executable instructions that are configured to carry out the various capabilities of the technical solutions described. For simplicity, the term "software program" is intended to encompass, at least, any one or more of the boundary generator 66, milling path generator 68, and/or geometric engine 72. The software program 80 can be implemented on the manipulator controller 60, navigation controller 62, or combination thereof.

One example of a system and method for generating the milling path is explained in U.S. Patent No. 9,119,655, entitled, "Surgical Manipulator Capable of Controlling a Surgical Tool in Multiple Modes," the disclosure of which is hereby incorporated by reference. In some examples, the virtual boundaries 70 and/or milling paths may be generated offline rather than on the manipulator computer 26 or navigation computer 36. Thereafter, the virtual boundaries 70 and/or milling paths may be utilized at runtime by the manipulator controller 60.

A clinical application 74 is provided to handle the user interaction. The clinical application 74 handles many aspects of user interaction and coordinates the surgical workflow, including pre-operative planning, implant placement, registration, bone preparation visualization, and post-operative evaluation of implant fit, etc. The clinical application 74 is configured to output to the displays 38. The clinical application 74 either may run on its own separate processor or may run alongside controller 62 on the navigation computer 36. In one example, the clinical application 74 interfaces with the path generator 68 and geometric engine 72 after implant placement is set by the user and then sends the tool path returned by the path generator 68 to controller 60 for execution. Manipulator controller 60 executes a tool path as described herein. The manipulator controller 60 may create certain segments (e.g., lead-in segments) when starting or resuming machining to smoothly get back to the generated tool path.

### II. Modifying a Bone Model

### a. Software Program Overview

Described herein are advanced techniques for modifying a bone model corresponding to a bone of the patient 12. Any of the techniques described herein can be implemented by the surgical system 10 and/or by the software program 80 executed by the surgical system 10. The software program 80 may be embodied by the clinical application 74 and/or the controller 30, or any part thereof. Additionally, the software program 80 may be embodied as a processor separate from the surgical system 10 described above (e.g., a processor of a separate computing device). Steps of the computer-implemented method of executing these techniques can be understood through description below.

The software program 80 may modify a bone model based on portions of the bone model to be disregarded during the planning of a surgical procedure. For example, the femoral head of a femur may not be relevant during the planning of a knee replacement surgery. In such instances, the software program 80 may modify a bone model corresponding to the femur to indicate portions of the bone model corresponding to the femoral head. As follows, during generation of the surgical plan for the knee replacement surgery, portions of the bone model corresponding to the femoral head may be disregarded. As another example, the distal tibia of a tibia may not be relevant during the planning of a knee replacement surgery. In such instances, the software program 80 may modify a bone model corresponding to the tibia to indicate portions of the bone model corresponding to the distal tibia. As follows, during generation of the surgical plan for the knee replacement surgery, portions of the bone model corresponding to the distal tibia may be disregarded. Advantageously, the software program 80 may provide a more accurate surgical plan by disregarding non-relevant portions of the bone model.

Additionally, or alternatively, the software program 80 may modify a bone model of a morbid bone based on a pre-morbid state of the morbid bone. In such instances, the software program 80 may predict/estimate the pre-morbid state of the morbid bone and modify the morbid bone model accordingly. For instance, the software program 80 may modify the morbid bone model by generating a pre-morbid bone model based on the predicted/estimated pre-morbid state. The software program 80 may then generate a surgical plan for the morbid bone based on the pre-morbid bone model. For example, the software program 80 may generate a milling path for the morbid bone based on the pre-morbid bone model. The robotic surgical system 10 may then prepare the morbid bone to receive an implant by milling the morbid bone in accordance with the generated milling path. Additionally, or alternatively, the software program 80 may predict/estimate and utilize the pre-morbid state to generate a treatment plan (e.g., a physical therapy treatment plan) for the morbid bone or to generate a diagnosis of the morbid bone (e.g., osteophytosis).

Advantageously, the software program 80 may provide a more accurate surgical plan by predicting a pre-morbid state of a morbid bone and modifying a corresponding morbid bone model accordingly. For instance, a morbid bone may include osteophytes and/or erosion, which may affect the surgical planning of an implant placement procedure, should the surgical plan be generated based on a morbid bone model of the morbid bone. By predicting a pre-morbid state of the bone, the software program 80 may generate a surgical plan to restore the morbid bone to the pre-morbid state. For instance, the software program 80 may generate a surgical plan for an implant placement procedure such that placement of the implant restores the morbid bone to the pre-morbid state.

### b. Identifying Portion of a Bone Model for Disregard

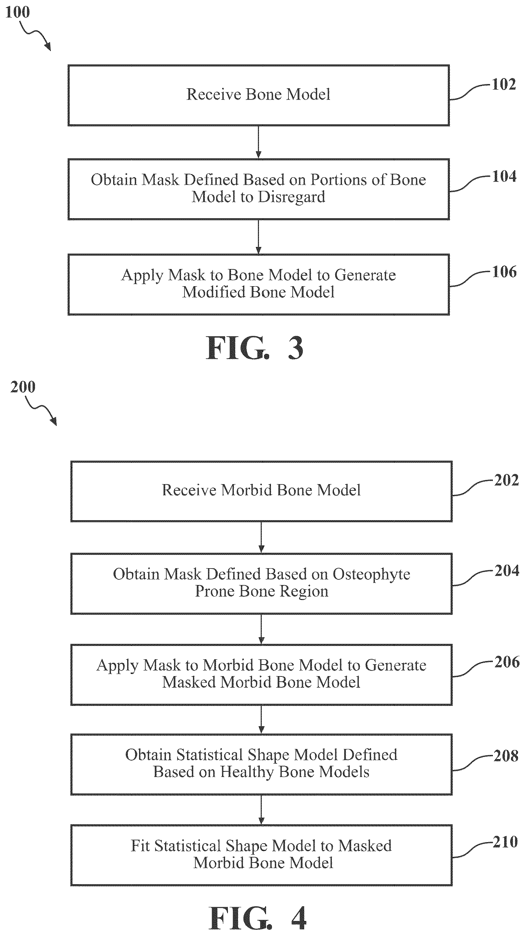

FIG. 3 illustrates a computer-implemented method 100 of identifying one or more portions of a bone model to be disregarded during surgical planning. The method 100 may be implemented by the previously described software program 80. As shown, the method 100 includes a step 102 of receiving a bone model of a bone; a step 104 of obtaining a mask defined based on portions of the bone model to be disregarded; and a step 106 of applying the mask to the bone model to generate a modified bone model.

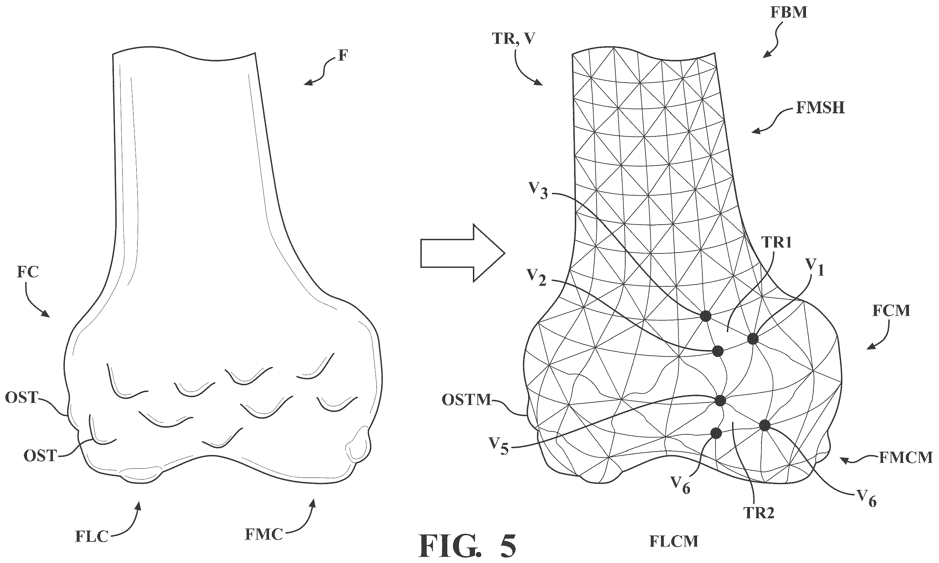

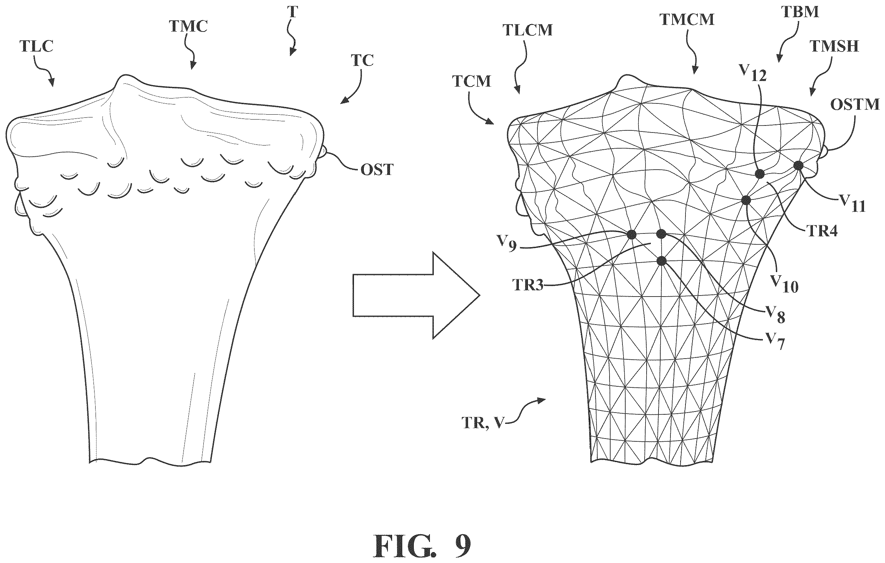

Example bone models that may be received by the software program 80 are shown in FIGS. 5 and 9. In the instance of FIG. 5, the bone model FBM is of a distal femur F of the patient 12. In the instance of FIG. 9, the bone model TBM is a bone model of a proximal tibia T of the patient 12. The received bone model may be a three-dimensional model generated using any suitable imaging technique. For example, the received bone model may be generated using X-ray imaging, 3D ultrasound imaging, magnetic resonance imaging (MRI), optical coherence tomography (OCT), computer tomography (CT), and/or positron emission tomography (PET). Additionally, the received bone model may be a bone model of any bone of the patient 12. For instance, the received bone model may be a bone model of a bone to be treated during a surgical procedure.

Referring to FIGS. 5 and 9, the bone models FBM, TBM include a mesh. As shown, the bone model FBM of FIG. 5 includes a mesh FMSH and the bone model TBM of FIG. 9 includes a mesh TMSH. As shown, each mesh FMSH, TMSH is formed of a plurality of triangles TR having vertices V. For example, referring to FIG. 5, triangles TR1, TR2 of the mesh FMSH are indicated. Triangle TR1 includes vertices V1-V3 and triangle TR2 includes vertices V4-V6. Similarly, referring to FIG. 9, triangles TR3, TR4 of the mesh TMSH are indicated. Triangle TR3 includes vertices V7-V9 and the triangle TR4 includes vertices V10-V12. Additionally, or alternatively, the mesh of a bone model may include polygons of any suitable shape and size. For example, the mesh may include any suitable combinations of triangles, rectangles, squares, pentagons, etc., for modeling a bone of a patient.

During step 104, the software program 80 obtains a mask defined based on portions of the bone model to be disregarded and, during step 106, the software program 80 applies the mask to generate a modified bone model. The modified bone model may indicate the portions of the bone model to be disregarded. For instance, the modified bone model may be embodied as the mesh of the bone model, but with the portions to be disregarded indicated. As will be described in greater detail below, the software program 80 may assign a weighting value to triangles TR and/or vertices V of the bone model to indicate the portions of the bone model to be disregarded. The modified bone model may also be embodied as the mesh of the bone model, but with the portions to be disregarded removed.

The mask may be embodied as list of triangles TR and/or vertices V of the bone model, or as a three-dimensional model including portions of the bone model. In an instance where the mask is embodied as a list, the listed triangles TR and/or vertices V may include corresponding portions of the bone model to be disregarded. In an instance where the mask is embodied as a three-dimensional model, the triangles TR and/or vertices V of the three-dimensional model may include the portions of the bone model to be disregarded.

The mask may include weighting values. In such instances, each weighting value may correspond to a triangle TR and/or vertex V of the bone model. The mask may be embodied as a list of weighting values. In an instance where the mask is embodied as a list of weighting values, each weighting value may correspond to a triangle TR and/or vertex V of the bone model to which the mask will be applied. Additionally, or alternatively, the mask may be embodied as a three-dimensional model, where each triangle TR and/or vertex V of the three-dimensional model is assigned a weighting value.

The weighting values of the mask may be selected to indicate portions of the bone model to disregard. For example, the weighting values may be binary numbers. In such instances, a weighting value of "1" may indicate a triangle TR and/or vertex V of the bone model to be preserved and a weighting value of "0" may indicate a triangle TR and/or vertex V to be disregarded. As another example, the weighting value may be any suitable number (e.g., any real or integer number). In such instances, a weighting value greater than a predetermined threshold value may indicate a triangle TR and/or vertex V of the bone model to be preserved and a weighting value less than a predetermined threshold value may indicate a triangle TR and/or vertex V to be disregarded. During step 106, the software program 80 may apply the mask to the bone model to generate the modified bone model by assigning the weighting values to the corresponding triangle TR and/or vertex V of the bone model. In some instances, the modified bone model includes the mesh of the bone model modified by the assigned weighting values.

In an example implementation, the mask may indicate portions of the bone model to be disregarded during planning of a knee replacement procedure. For example, a bone model of a femur may include the proximal femur, which may be disregarded during the knee replacement procedure. In such instances, during step 104, the mask may indicate the triangles TR and/or vertices V corresponding to the proximal femur. During step 106, the software program 80 may apply the mask to the bone model of the femur to generate a modified bone model of the femur, where the modified bone model indicates the triangles TR and/or vertices V corresponding to the proximal femur. As another example, a bone model of a tibia may include a distal tibia, which may be disregarded during the knee replacement procedure. In such instances, during step 104, the mask may indicate the triangles TR and/or vertices V corresponding to the distal tibia. During step 106, the software program 80 may apply the mask to the bone model of the tibia to generate a modified bone model of the tibia, where the modified bone model indicates the triangles TR and/or vertices V corresponding to the distal tibia.

In some instances, the mask may be generated or selected based on the surgical procedure. For example, in an instance where the bone model is a bone model of a femur and the surgical procedure is a total knee replacement procedure, the software program 80 may select a predetermined mask or generate a mask that indicates the triangles TR and/or vertices V corresponding to the proximal femur. The selected/generated mask may include a list of weighting values corresponding to triangles TR and/or vertices V of the bone model. The weighting values may be selected/generated by the software program 80. In one implementation, a user may select a surgical procedure using the clinical application 74 and the software program 80 may generate the mask or select a predetermined mask based on the selection.

In some instances, the mask may be generated based on a user input. For example, a user may interact with the bone model via the clinical application 74 to indicate which portions of the bone model should be disregarded during surgical planning (e.g., using a cursor provided by a user interface of the clinical application 74). The software program 80 may then generate the mask based on the portions of the bone model indicated by the user. For example, the generated mask may include a list of weighting values corresponding to triangles TR and/or vertices V of the bone model. As another example, a user may select a predetermined mask using the clinical application 74.

### c. Generating a Pre-Morbid State of a Morbid Bone

FIG. 4 illustrates a computer-implemented method 200 of predicting a pre-morbid state of a morbid bone. Specifically, the method 200 predicts the pre-morbid state by receiving a morbid bone model corresponding to the morbid bone and predicting a pre-morbid state of the morbid bone model. The method 200 may be implemented by the previously described software program 80. As shown, the method 200 includes a step 202 of receiving a morbid bone model of a morbid bone; a step 204 of obtaining a mask defined based on a bone region statistically identified as being osteophyte prone; a step 206 of applying the mask to the morbid bone model to generate a masked morbid bone model; a step 208 of obtaining a statistical shape model defined based on healthy bone models; and a step 210 of fitting the statistical shape model to the masked morbid bone model to generate a pre-morbid bone model to predict the pre-morbid state of the morbid bone model.

A morbid bone may be defined as any bone including unhealthy characteristics. For instance, a morbid bone may include osteophytes and/or erosion where the osteophytic portion and the eroded portion may be defined as a morbid portion of the bone. Referring to FIG. 5, a morbid distal femur F is shown. As shown, the morbid distal femur F includes osteophytes OST located at a condylar region FC of the femur F. Specifically, the morbid distal femur F includes osteophytes OST disposed on the medial condyle FMC and the lateral condyle FLC of the morbid distal femur F. Referring to FIG. 9, a morbid proximal tibia T is shown. As shown, the morbid proximal tibia T includes osteophytes OST located at a condylar region TC of the proximal tibia T. Specifically, the morbid proximal tibia T includes osteophytes OST disposed on the medial condyle TMC and the lateral condyle TLC of the morbid proximal tibia T.

The method 200 may be applied for any bone including a morbid portion. In the examples described herein, the morbid portion is characterized as an osteophytic portion of the bone and the method 200 is applied to a morbid bone model including the osteophytic portion. However, it is contemplated that the method 200 may be similarly applied to a morbid bone model including an eroded portion.

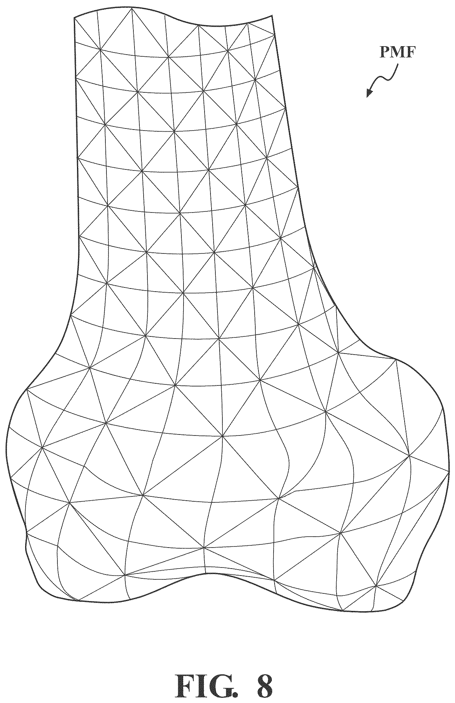

FIGS. 5 and 9 illustrate the step 202 of receiving a morbid bone model of a morbid bone. In the instance of FIG. 4, the morbid bone model FBM is a bone model of the morbid distal femur F of the patient 12. In the instance of FIG. 8, the morbid bone model TBM is a bone model of a morbid distal tibia T of the patient 12. As shown, the morbid femur bone model FBM and the morbid tibia bone model TBM include model osteophytes OSTM, which model the osteophytes OST of the morbid distal femur F and the morbid proximal tibia T. Additionally, the morbid femur bone model FBM includes a model condylar region FCM modeling the condylar region FC of the morbid distal femur F, a model medial condyle FMCM modeling the medial condyle FMC of the morbid distal femur F, and a model lateral condyle FLCM modeling the lateral condyle FLC of the morbid distal femur F. The morbid tibia bone model TBM includes a model condylar region TCM modeling the condylar region TC of the morbid proximal tibia T, a model medial condyle TMCM modeling the medial condyle TMC of the morbid proximal tibia T, and a model lateral condyle TLCM modeling the lateral condyle TLC of the morbid proximal tibia T.

The received morbid bone model may be a three-dimensional model generated using any suitable imaging technique. For example, the received morbid bone model may be generated using X-ray imaging, 3D ultrasound imaging, magnetic resonance imaging (MRI), optical coherence tomography (OCT), computer tomography (CT), and/or positron emission tomography (PET). Additionally, the received morbid bone model may be a morbid bone model of any morbid bone of the patient 12. For instance, the received morbid bone model may be a morbid bone model of a morbid bone to be treated during a surgical procedure.

Referring to FIGS. 5 and 9, the morbid bone models FBM, TBM include a mesh. As shown, the morbid femur bone model FBM of FIG. 5 includes a mesh FMSH and the morbid tibia bone model TBM of FIG. 9 includes a mesh TMSH. As shown, each mesh FMSH, TMSH is formed of a plurality of triangles TR having vertices V. For example, referring to FIG. 5, triangles TR1, TR2 of the mesh FMSH are indicated. Triangle TR1 includes vertices V1-V3 and triangle TR2 includes vertices V4-V6. Similarly, referring to FIG. 9, triangles TR3, TR4 of the mesh TMSH are indicated. Triangle TR3 includes vertices V7-V9 and the triangle TR4 includes vertices V10-V12. Additionally, or alternatively, the mesh of a morbid bone model may include polygons of any suitable shape and size. For example, the mesh may include any suitable combinations of triangles, rectangles, squares, pentagons, etc., for modeling a morbid bone of a patient.

Generally, the mask includes, as a list or as a three-dimensional model (in accordance with the previously described mask of the method 100), portions of the morbid bone model including morbid conditions. In some instances, the mask may be defined based on a bone region statistically identified as being prone to morbid conditions. As used herein, a bone region that is "statistically identified as being osteophyte prone" can refer to a region determined based on analysis of aggregated clinical data across a population, indicating an increased likelihood of osteophyte/morbidity relative to other (healthy) regions of the bone. For example, the mask may be defined based on a bone region statistically identified as being erosion prone. As another example, the mask may be defined based on a bone region statistically identified as being osteophyte prone. In some instances, a software program (e.g., the software program 80 or a separate software program) may generate the mask based on determining a bone region statistically identified as being prone to morbid conditions. For example, the software program may evaluate a dataset, such as a clinical dataset, including morbid bone models and determine the bone region statistically identified as being prone to morbid conditions based on the evaluation. In some instances, the software program may generate the mask based on receiving information regarding a bone region statistically identified as being prone to morbid conditions. In some instances, a user may indicate a bone region of a morbid bone model that includes a morbid condition (e.g., erosion and/or osteophytes) via the clinical application 74. For instance, the user may interact with the bone model via a user interface of the clinical application 74 (e.g., using a cursor provided by a user interface of the clinical application 74) to indicate a bone region of the morbid bone model including a morbid condition. The software program 80 may then generate the mask based on the user's indication. In some instances, the mask may be generated or selected based on the surgical procedure, in accordance with above description of method 100. The mask can be predetermined, preoperatively determined, intraoperatively determined, or dynamically determined. The statistical identification of morbid-prone regions may be based on any combination of clinical imaging data, patient outcome data, annotated anatomical models, or retrospective analyses, and does not require a particular statistical technique, data modality, or sample size.

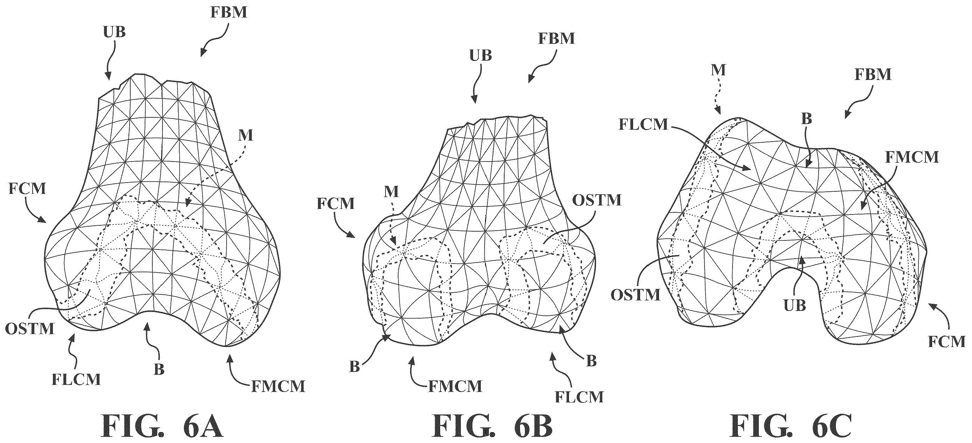

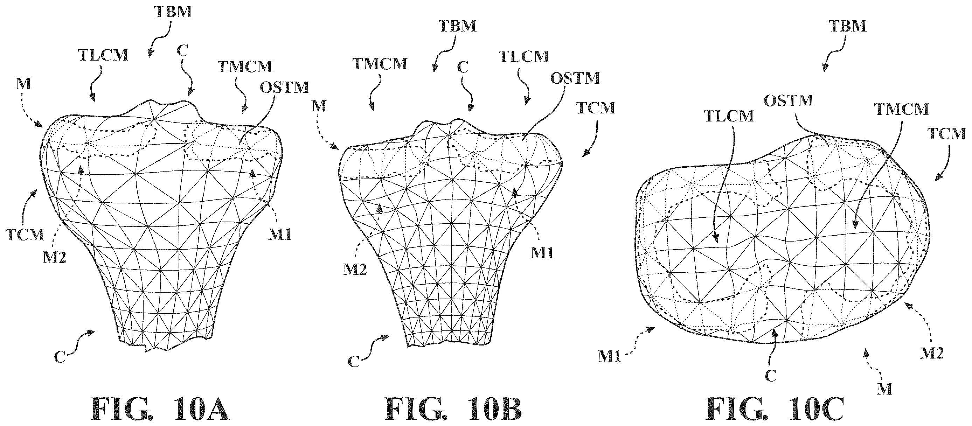

During step 204, the software program 80 obtains the mask. An illustration of an exemplary mask M is shown in FIGS. 6A-6C, where the mask M is defined based on a bone region of the femur F statistically identified as being osteophyte prone. An illustration of an exemplary mask M is also shown in FIGS. 10A-10C, where the mask M is defined based on a bone region of the tibia T statistically identified as being osteophyte prone. The masks M of FIGS. 6A-6C and 10A-10C are shown relative to the morbid femur bone model FBM and the morbid tibia bone model TBM, respectively, to illustrate that the mask includes regions of the morbid femur bone model FBM and the morbid tibia bone model TBM including model osteophytes OSTM. As shown, the model osteophytes OSTM are located within the mask M. More particularly, the mask of FIGS. 6A-6C includes, as a list or as a three-dimensional model, the triangles TR, and/or vertices V of the morbid femur bone model FBM that include the model osteophytes OSTM. Similarly, the mask of FIGS. 10A-10C includes, as a list or as a three-dimensional model, the triangles TR, and/or vertices V of the morbid tibia bone model TBM that include the model osteophytes OSTM.

Referring to FIGS. 6A-6C, the mask M is defined based on the condylar region FC of the morbid distal femur F statistically identified as having osteophytes OST. Specifically, the mask M is defined as outlining a periphery of the medial condyle FMC and the lateral condyle FLC of the morbid distal femur F. As shown in FIGS. 6A-6C, the mask M outlines a periphery of the model medial condyle FMCM and the model lateral condyle FLCM. More particularly, the mask of FIGS. 6A-6C includes, as a list or as a three-dimensional model, the triangles TR, and/or vertices V of the morbid femur bone model FBM that outline a periphery of the model medial condyle FMCM and the model lateral condyle FLCM.

Referring to FIGS. 10A-10C, the mask M is defined based on the condylar region TC of the morbid proximal tibia T statistically identified as having osteophytes OST. Specifically, the mask M comprises a first portion M1 configured to outline a periphery of the lateral condyle TLC of the morbid proximal tibia T and a second portion M2 configured to outline a periphery of the medial condyle TMC of the morbid proximal tibia T. As shown in FIGS. 10A-10C, the first portion M1 outlines a periphery of the model lateral condyle TLCM and the second portion M2 outlines a periphery of the model medial condyle TLCM. More particularly, the mask of FIGS. 10A-10C includes, as a list or as a three-dimensional model, the triangles TR, and/or vertices V of the morbid femur bone model FBM that outline a periphery of the model lateral condyle TLCM and outline a periphery of the medial condyle TMC.

In other instances, the mask may include any suitable shape. More particularly, the triangles TR and/or vertices V of the morbid bone model indicated by the mask as a list or included by the mask as a three-dimensional model may form any suitable shape. For example, the mask may include an open or closed-off shape. As an example, the mask M of FIGS. 6A-6C includes a closed-off shape, where the closed-off shape of the mask M connects with itself forming a bounded portion B and an unbounded portion UB. As another example, the mask M of FIGS. 10A-10C includes an open shape, where the open shape of the mask M does not connect with itself. Specifically, the first portion M1 and the second portion M2 forming a continuous portion C.

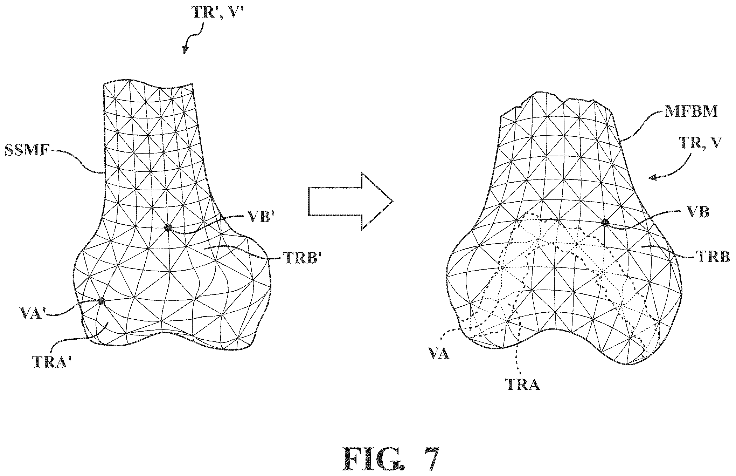

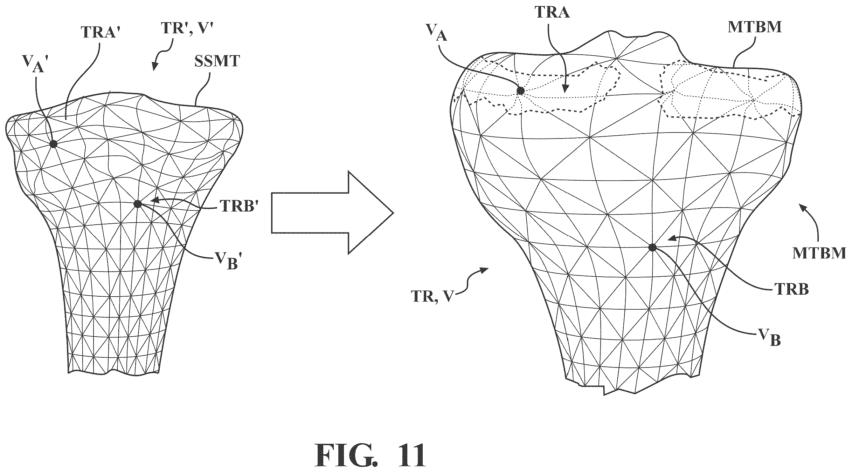

During step 206, the software program 80 applies the mask to the morbid bone model to generate a masked morbid bone model. Example masked morbid bone models MFBM, MTBM are shown in FIGS. 7 and 11. During step 206, the software program 80 may apply the mask M of FIGS. 6A-6C to the morbid femur bone model FBM to generate the masked morbid femur bone model MFBM of FIG. 7. s another example, during step 206, the software program 80 may apply the mask M of FIGS. 10A-10C to the morbid tibia bone model TBM to generate the masked morbid tibia bone model MTBM. The masked morbid bone model may be embodied as the mesh of the morbid bone model, but with the portions including morbid conditions removed. More particularly, during step 206, the software program 80 may apply the mask to the morbid bone model to generate the masked morbid bone model, where the masked morbid bone model includes the mesh of the morbid bone model with the portions including morbid conditions removed. For example, referring to FIGS. 6A-6C, the software program 80 may remove portions of the morbid femur bone model FBM included by the mask M and preserve the bounded and unbounded portions B, UB of the morbid femur bone model FBM to generate the masked morbid femur bone model during step 206. As another example, referring to FIGS. 10A-10C, the software program 80 may remove portions of the morbid tibia bone model TBM included by the mask M and preserve the continuous portion C of the morbid tibia bone model TBM to generate the masked morbid tibia bone model during step 206. Such preservation can retain connectivity while allowing replacement inside the closed shape.

The masked morbid bone model may also be embodied as the mesh of the morbid bone model, but with triangles TR and/or vertices V of the morbid bone model modified by assigned weighting values. More particularly, during step 206, the software program 80 may apply the mask to the morbid bone model to generate the masked morbid bone model, where the masked morbid bone model includes the mesh of the morbid bone model modified by assigned weighting values. In accordance with the previously described mask of the method 100, the weighting values may be a binary number ("1" or "0") or any real or integer number. For example, in the instances of FIGS. 7 and 11, the dotted vertex VA may be assigned a "0" and the solid vertex VB may be assigned a "1".

The software program 80 may fit a statistical shape model to the masked morbid bone model to generate the pre-morbid bone model to predict the pre-morbid state of the morbid bone model. In an instance where the masked morbid bone model includes the mesh of the morbid bone model with the portions including morbid conditions removed, the removed portions of the masked morbid bone model may not affect the fitting of the statistical shape model. In such instances, the software program 80 may adjust a triangle or a vertex of the statistical shape model to fit the statistical shape model to the unremoved portions of the masked morbid bone model. In an instance where the masked morbid bone model includes the mesh of the morbid bone model modified by assigned weighting values, the software program 80 may fit the statistical shape model to the masked morbid bone model based on the weighting value. In such instances, the software program 80 may adjust a triangle or a vertex of the statistical shape model to fit the statistical shape model to the masked morbid bone model based on the weighting value of the corresponding triangle TR or vertex V of the masked morbid bone model. As an example, the greater the weighting value assigned to a triangle TR or vertex V of the masked morbid bone model, the greater the adjustment of the corresponding triangle or vertex of the statistical shape model. For example, in the instances of FIGS. 7 and 11, the solid vertex VB may be assigned a greater number than the dotted vertex VA.

During step 208, the software program 80 obtains a statistical shape model defined based on health bone models. The statistical shape model may be generated by the software program 80 or a separate software program. For example, the software program may evaluate a dataset including healthy bone models to generate the statistical shape model. Example statistical shape models SSMF, SSMT are shown in FIGS. 7 and 11. A statistical shape model SSMF of a healthy distal femur is shown in FIG. 7. A statistical shape model SSMT of a healthy proximal tibia is shown in FIG. 10.

The statistical shape model may include a mesh formed of a plurality of triangles TR' and vertices V.' For instance, referring to FIGS. 7 and 11, triangles TRA', TRB' of the plurality of triangles TR' of the statistical shape models SSMF, SSMT are indicated and vertices VA', VB' of the plurality of vertices V of the statistical shape models SSMF, SSMT are indicated. Each of the plurality of triangles TR' and vertices V' correspond to a triangle TR and vertex V of the masked morbid bone model. For example, in FIG. 7, triangle TRA' of the statistical shape model SSMF corresponds to triangle TRA of the masked morbid bone model MFBM and triangle TRB' of the statistical shape model SSMF corresponds to triangle TRB of the masked morbid bone model MFBM. Additionally, vertex VA' of the statistical shape model SSMF corresponds to vertex VA of the masked morbid bone model MFBM and vertex VB' of the statistical shape model SSMF corresponds to vertex VB of the masked morbid bone model MFBM. In FIG. 11, triangle TRA' of the statistical shape model SSMT corresponds to triangle TRA of the masked morbid bone model MTBM and triangle TRB' of the statistical shape model SSMT corresponds to triangle TRB of the masked morbid bone model MTBM. Additionally, vertex VA' of the statistical shape model SSMT corresponds to vertex VA of the masked morbid bone model MTBM and vertex VB' of the statistical shape model SSMT corresponds to vertex VB of the masked morbid bone model MTBM.

During step 210, the statistical shape model is fitted to the masked morbid bone model to generate the pre-morbid bone model to predict the pre-morbid state of the morbid bone model. For example, referring to FIG. 7, the statistical shape model SSMF of a healthy distal femur is fitted to the masked morbid bone model MFBM. Referring to FIG. 11, the statistical shape model SSMT of a healthy proximal tibia is fit to the masked morbid bone model MTBM.

The software program 80 may adjust a size and shape of the statistical shape model based on a size and shape of the masked morbid bone model to fit the statistical shape model to the masked morbid bone model. The software program 80 may adjust a position of one or more triangles TR' and/or one or more vertices V' of the statistical shape model to adjust a size and shape of the statistical shape model and to fit the statistical shape model to the masked morbid bone model. For instance, the software program 80 may adjust a position of a triangle TR' and/or vertex V' of the statistical shape model in a coordinate system of the statistical shape model based on a position of a corresponding triangle TR and/or vertex V of the masked morbid bone model in a coordinate system of the masked morbid bone model. In a more specific instance, the software program 80 may adjust a position of a triangle TR' and/or vertex V' of the statistical shape model in a coordinate system of the statistical shape model to match a position of a corresponding triangle TR and/or vertex V of the masked morbid bone model in a coordinate system of the masked morbid bone model.

In an instance where the masked morbid bone model includes the mesh of the morbid bone model with the portions including morbid conditions removed, the removed portions of the masked morbid bone model may not affect the fitting of the statistical shape model. In such instances, the software program 80 may adjust a triangle or a vertex of the statistical shape model to fit the statistical shape model to the unremoved portions of the masked morbid bone model. The software program 80 may adjust a triangle TR' and/or vertex V' of the statistical shape model corresponding to a removed triangle TR and/or vertex V of the masked morbid bone model based on an adjustment made to a proximate triangle TR' and/or vertex V' corresponding to an unremoved triangles TR and/or vertices V of the masked morbid bone model. In one such instance, the software program 80 may adjust a triangle TR' and/or vertex V' of the statistical shape model corresponding to a removed triangle TR and/or vertex V based on an average of adjustment made to more than one proximate triangle TR' and/or vertex V' corresponding to an unremoved triangles TR and/or vertices V of the masked morbid bone model.

In an instance where the masked morbid bone model includes the mesh of the morbid bone model modified by assigned weighting values, the software program 80 may fit the statistical shape model to the masked morbid bone model based on the weighting value. The software program 80 may adjust a position of a triangles TR' and/or vertex V' of the statistical shape model based on the weighting value assigned to the corresponding triangle TR and/or vertex V of the masked morbid bone model.

In instances where the weighting value is a binary number, the software program 80 may adjust a position of a triangle TR' and/or vertex V' based on whether the corresponding triangle TR and/or vertex V is assigned a "1" or a "0". For example, the software program 80 may adjust a position of a triangle TR' and/or vertex V' of the statistical shape model in a coordinate system of the statistical shape model to match a position of a corresponding triangle TR and/or vertex V of the masked morbid bone model in a coordinate system of the masked morbid bone model if the weighting value of the triangle TR and/or vertex V is "1". Additionally, the software program 80 may not adjust a position of a triangle TR' and/or vertex V' of the statistical shape model if the weighting value of the triangle TR and/or vertex V is "0". For example, in the instances of FIGS. 7 and 11, the dotted vertex VA may be assigned a "0" and the solid vertex VB may be assigned a "1". As follows, the software program 80 may adjust a position of the vertex VB' corresponding to dotted vertex VB and the software program 80 may not adjust a position of vertex VA' corresponding to solid vertex VA during step 210.

In instances where the weighting value is a decimal or whole number, the software program 80 may determine an amount of adjustment for a triangle TR' and/or vertex V' based on a magnitude of the weighting value assigned to the corresponding triangle TR and/or vertex V. For instance, the greater the weighting value of a triangle TR and/or vertex V, the greater the amount of adjustment of the corresponding triangle TR' and/or vertex V.' In such instances, a maximum amount of adjustment may be determined by a position of the triangle TR and/or vertex V in the coordinate system of the masked morbid bone model. For instance, the software program 80 may adjust a position of a triangle TR' and/or vertex V' in the coordinate system of the statistical shape model such that the position does not exceed a position of a corresponding triangle TR and/or vertex V of the masked morbid bone model in a coordinate system of the masked morbid bone model. For example, in the instances of FIGS. 7 and 11, the solid vertex VB may be assigned a greater number than the solid vertex VB. As follows, the software program 80 may provide a greater amount of adjustment to vertex VB' corresponding to dotted vertex VB than to vertex VA' corresponding to solid vertex VA during step 210.

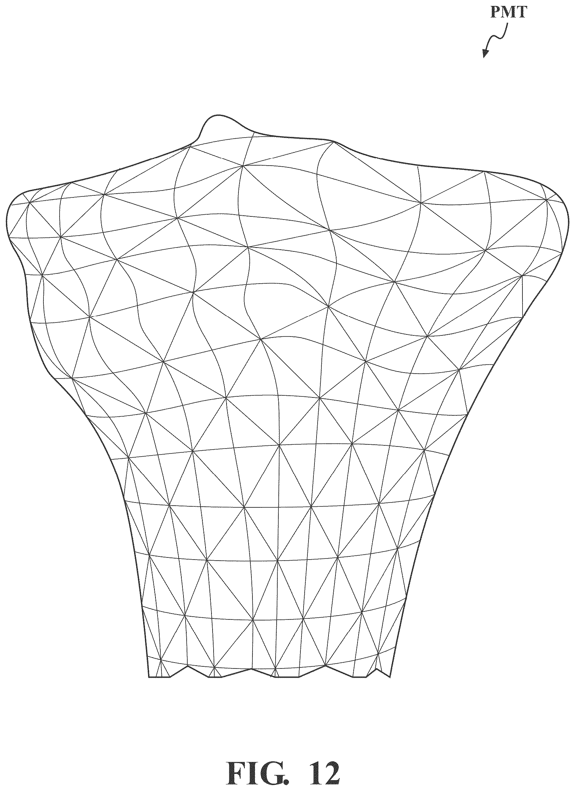

As previously stated, during step 210, the statistical shape model is fit to the masked morbid bone model to generate a pre-morbid bone model. Example pre-morbid bone models PMF, PMT are shown in FIGS. 8 and 12, respectively. The pre-morbid bone model PMF of FIG. 8 illustrates a pre-morbid state of the morbid femur bone model FBM. The pre-morbid bone model PMT of FIG. 8 illustrates a pre-morbid state of the morbid tibia bone model TBM. As shown, the pre-morbid bone models PMF, PMT do not include the morbid portions of the morbid bone models FBM, TBM (shown in FIGS. 5 and 9), respectively. Specifically, the pre-morbid bone models PMF, PMT do not include the model osteophytes OSTM of the morbid bone models FBM, TBM.

The pre-morbid bone model may be formed of the fitted statistical shape model. In such instances, the software program 80 may fit the statistical shape model to the masked morbid bone model and the fitted statistical shape model serves as the pre-morbid bone model.

The pre-morbid bone model may be formed in-part by the fitted statistical shape model and formed in-part by the masked morbid bone model. As an example, in an instance where the masked morbid bone model includes the mesh of the morbid bone model with the portions including morbid conditions removed, the fitted statistical shape model may replace the removed portions of the masked morbid bone model. Additionally, the pre-morbid bone model may include the unremoved portions of the masked morbid bone model. As another example, in an instance where the software program 80 applies the mask to the morbid bone model to generate the masked morbid bone model and the masked morbid bone model includes the mesh of the morbid bone model modified by assigned weighting values, the fitted statistical shape model may replace the portions of the mesh of the morbid bone model modified by assigned weighting values. Additionally, the pre-morbid bone model may include the unmodified portions of the masked morbid bone model.

The disclosed techniques provide numerous technical solutions to limitations associated with anatomically modeling morbid bones by reducing or eliminating the influence of pathological bone regions during model generation. Rather than relying solely on a model of the bone in its current, morbid state, the disclosed approaches identify osteophyte-prone or otherwise non-representative regions using population-derived statistical information and selectively modulate, remove, or replace those regions during anatomical modeling. By fitting a statistical shape model, defined based on healthy bone anatomy, to preserved portions of the morbid bone model that more reliably reflect the underlying anatomical structure, the disclosed techniques generate an estimated pre-morbid bone model that more accurately represents the bone's baseline anatomical state. This technical approach improves the robustness, consistency, and anatomical relevance of computer-generated bone models, particularly in the presence of osteophytes or degenerative changes, and enables downstream computational processes, such as surgical planning, implant positioning, alignment determination, and anatomical analysis, to be performed based on geometries that are less affected by pathological artifacts. As a result, the disclosed techniques provide improved computational modeling of anatomical structures and enhanced technical support for clinical planning workflows.

Several embodiments have been described in the foregoing description. However, the embodiments discussed herein are not intended to be exhaustive or limit the invention to any particular form. The terminology, which has been used, is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

The following CLAUSES define example implementations of the disclosed subject matter, with various features and combinations described herein being applicable individually or in combination, unless expressly stated otherwise:
Clause 1A. A computer program product (80) comprising instructions, which are executable by one or more processors, to: receive a morbid bone model (FBM, TBM); obtain a mask (M) defined based on a bone region statistically identified as being osteophyte-prone; apply the mask (M) to the morbid bone model (FBM, TBM) to remove portions of the morbid bone model (FBM, TBM) and to generate a masked morbid bone model (MFBM, MTBM); obtain a statistical shape model (SSMF, SSMT) defined based on healthy bone models; and fit the statistical shape model (SSMF, SSMT) to the masked morbid bone model (MFBM, MTBM) to generate a pre-morbid bone model (PMF, PMT) representing an estimated pre-morbid state of the morbid bone model (FBM, TBM). Clause 2A. The computer program product (80) of any preceding clause, wherein the pre-morbid bone model (PMF, PMT) is formed of the fitted statistical shape model (SSMF, SSMT). Clause 3A. The computer program product (80) of any preceding clause, wherein the pre-morbid bone model (PMF, PMT) is formed in-part by the fitted statistical shape model (SSMF, SSMT) and formed in-part by the masked morbid bone model (MFBM, MTBM). Clause 4A. The computer program product (80) of any preceding clause, wherein the masked morbid bone model (MFBM, MTBM) includes unremoved portions of the morbid bone model (FBM, TBM) and excludes the removed portions of the morbid bone model (FBM, TBM), and wherein the fitted statistical shape model (SSMF, SSMT) replaces the removed portions of the morbid bone model (FBM, TBM) to generate the pre-morbid bone model (PMF, PMT). Clause 5A. The computer program product (80) of any preceding clause, wherein the instructions are executable by one or more processors to remove portions of the morbid bone model (FBM, TBM) within a geometry of the mask (M), while preserving portions of the morbid bone model (FBM, TBM) outside the geometry of the mask (M). Clause 6A. The computer program product (80) of any preceding clause, wherein: the morbid bone model (FBM, TBM) is a bone model of a distal femur; the mask (M) is defined based on a condylar region of the distal femur statistically identified as being osteophyte prone; and the mask (M) is further defined as outlining a periphery of a medial condyle and a lateral condyle of the distal femur. Clause 7A. The computer program product (80) of clause 6A, wherein the mask (M) comprises a closed-off shape and wherein the instructions are executable by one or more processors to: apply the mask (M) to the morbid bone model (FBM, TBM) to create: a bounded portion of the morbid bone model (FBM, TBM) bounded by the closed-off shape of the mask (M), and an unbounded portion of the morbid bone model (FBM, TBM) adjacent to the closed-off shape of the mask (M); and preserve the bounded and unbounded portions of the morbid bone model (FBM, TBM) to generate the masked morbid bone model (MFBM, MTBM). Clause 8A. The computer program product (80) of any preceding clause, wherein: the morbid bone model (FBM, TBM) is a bone model of a proximal tibia; the mask (M) is defined based on a condylar region of the proximal tibia statistically identified as being osteophyte prone; and the mask (M) comprises a first portion configured to outline a periphery of a lateral condyle of the proximal tibia, and a second portion configured to outline a periphery of a medial condyle of the proximal tibia. Clause 9A. The computer program product (80) of clause 8A, wherein the first portion and the second portion of the mask (M) each comprise an open shape and wherein the instructions are executable by one or more processors to: apply the mask (M) to the morbid bone model (FBM, TBM) to create a continuous portion of the morbid bone model (FBM, TBM) and preserve the continuous portion of the morbid bone model (FBM, TBM) to generate the masked morbid bone model (MFBM, MTBM). Clause 10A. The computer program product (80) of any preceding clause, wherein fitting the statistical shape model (SSMF, SSMT) to the masked morbid bone model (MFBM, MTBM) comprises adjusting one or more shape parameters of the statistical shape model (SSMF, SSMT) to reduce a difference between the statistical shape model (SSMF, SSMT) and preserved portions of the masked morbid bone model (MFBM, MTBM), while excluding the removed portions from influencing the fitting. Clause 11A. The computer program product (80) of any preceding clause, wherein the mask (M) is generated by analyzing aggregated clinical data across a population of subjects to identify anatomical regions of the bone exhibiting a statistically increased likelihood of osteophyte formation. Clause 12A. The computer program product (80) of any preceding clause, wherein the statistical shape model (SSMF, SSMT) is selected from a plurality of statistical shape models based on an anatomical similarity between the morbid bone model (FBM, TBM) and healthy bone models used to define each statistical shape model (SSMF, SSMT). 13A. The computer program product (80) of any preceding clause, wherein the pre-morbid bone model (PMF, PMT) is utilized to automatically generate one or more parameters of a surgical or treatment plan derived from the estimated pre-morbid state of the morbid bone model (FBM, TBM). Clause 14A. A computer-implemented method (100, 200) for implementing the instructions of any preceding clause.
Clause 1B. A computer program product (80) comprising instructions, which are executable by one or more processors, to: receive a bone model including a mesh (FMSH, TMSH) formed of a plurality of triangles (TR) having vertices (V); obtain a mask (M) defined based on vertices (V) of the bone model to be disregarded, wherein the mask (M) includes a plurality of weighting values corresponding to the vertices (V) of the bone model to be disregarded; and apply the mask (M) to the bone model to generate a modified bone model by assigning the weighting values to the corresponding vertices (V) of the bone model, wherein the modified bone model includes the mesh (FMSH, TMSH) of the bone model modified by the assigned weighting values. Clause 2B. The computer program product (80) of any preceding clause, wherein the weighting values are configured to reduce an influence of the corresponding vertices (V) during subsequent processing of the modified bone model. Clause 3B. The computer program product (80) of any preceding clause, wherein weighting values assigned to vertices (V) to be disregarded are lower than weighting values assigned to vertices (V) not to be disregarded. Clause 4B. The computer program product (80) of any preceding clause, wherein weighting values include zero or near-zero values corresponding to vertices (V) of the bone model to be disregarded. Clause 5B. The computer program product (80) of any preceding clause, wherein vertices (V) of the bone model not associated with the mask (M) are assigned weighting values that preserve their influence in the modified bone model. Clause 6B. The computer program product (80) of any preceding clause, wherein the mask (M) is defined based on a bone region statistically identified as being osteophyte-prone. Clause 7B. The computer program product (80) of any preceding clause, wherein the mask (M) is generated dynamically for a specific bone model during execution of the instructions. Clause 8B. The computer program product (80) of any preceding clause, wherein the bone model represents a morbid bone. Clause 9B. The computer program product (80) of clause 8B, wherein the morbid bone model (FBM, TBM) represents a distal femur. Clause 10B. The computer program product (80) of clause 8B, wherein the morbid bone model (FBM, TBM) represents a proximal tibia. Clause 11B. The computer program product (80) of any preceding clause, wherein each weighting value corresponds to a respective vertex (V) of the mesh (FMSH, TMSH). Clause 12B. The computer program product (80) of any preceding clause, wherein applying the mask (M) does not remove vertices (V) from the mesh (FMSH, TMSH). Clause 13B. The computer program product (80) of any preceding clause, wherein the modified bone model is configured to be used to fit a statistical shape model (SSMF, SSMT), wherein the statistical shape model (SSMF, SSMT) is defined based on healthy bone models. Clause 14B. The computer program product (80) of any preceding clause, wherein the modified bone model is used to generate a pre-morbid bone model (PMF, PMT). Clause 15B. A computer-implemented method (100, 200) for implementing the instructions of any preceding clause.

## Claims

1. A computer program product (80) comprising instructions that are executable by one or more processors to:
receive a morbid bone model (FBM, TBM);
obtain a mask (M) defined based on a bone region statistically identified as being osteophyte prone;
apply the mask (M) to the morbid bone model (FBM, TBM) to generate a masked morbid bone model (MFBM, MTBM);
obtain a statistical shape model (SSMF, SSMT) defined based on healthy bone models; and
fit the statistical shape model (SSMF, SSMT) to the masked morbid bone model (MFBM, MTBM) to generate a pre-morbid bone model (PMF, PMT) representing an estimated pre-morbid state of the morbid bone model (FBM, TBM).

2. The computer program product (80) of claim 1, wherein the statistical shape model (SSMF, SSMT) is further defined as being trained on healthy bone models.

3. The computer program product (80) of any preceding claim, wherein the pre-morbid bone model (PMF, PMT) is formed of the fitted statistical shape model (SSMF, SSMT).

4. The computer program product (80) of any preceding claim, wherein the pre-morbid bone model (PMF, PMT) is formed in-part by the fitted statistical shape model (SSMF, SSMT) and formed in-part by the masked morbid bone model (MFBM, MTBM).

5. The computer program product (80) of any preceding claim, wherein:
the morbid bone model (FBM, TBM) comprises a mesh (FMSH, TMSH) formed of a plurality of triangles (TR) having vertices (V);
the mask (M) includes a plurality of weighting values corresponding to at least some vertices (V) of the morbid bone model (FBM, TBM); and
the instructions are executable by one or more processors to apply the mask (M) by being configured to assign the weighting values to the corresponding vertices (V) of the morbid bone model (FBM, TBM), and wherein the masked morbid bone model (MFBM, MTBM) includes the mesh (FMSH, TMSH) of the morbid bone model (FBM, TBM) modified by the assigned weighting values.

6. The computer program product (80) of claim 5, wherein the instructions are executable by one or more processors to adjust a size and shape of the statistical shape model (SSMF, SSMT) based on a size and shape of the masked morbid bone model (MFBM, MTBM) to fit the statistical shape model (SSMF, SSMT) to the masked morbid bone model (MFBM, MTBM).

7. The computer program product (80) of claim 5, wherein:
the statistical shape model (SSMF, SSMT) comprises a mesh (FMSH, TMSH) formed of a plurality of triangles (TR) having vertices (V);
each of the plurality of vertices (V) of the masked morbid bone model (MFBM, MTBM) corresponds to a vertex (V) of the statistical shape model (SSMF, SSMT); and
the instructions are executable by one or more processors to adjust a position of the plurality of vertices (V) of the statistical shape model (SSMF, SSMT) based on the weighting values of the plurality of vertices (V) of the masked morbid bone model (MFBM, MTBM) to adjust a size and shape of the statistical shape model (SSMF, SSMT).

8. The computer program product (80) of any preceding claim, wherein:
the morbid bone model (FBM) is a bone model of a distal femur; and
the mask (M) is defined based on a condylar region of the distal femur statistically identified as being osteophyte prone.

9. The computer program product (80) of claim 8, wherein the mask (M) is further defined as outlining a periphery of a medial condyle and a lateral condyle of the distal femur.

10. The computer program product (80) of any preceding claim, wherein:
the morbid bone model (TBM) is a bone model of a proximal tibia; and
the mask (M) is defined based on a condylar region of the proximal tibia statistically identified as being osteophyte prone.

11. The computer program product (80) of claim 10, wherein the mask (M) is further defined as outlining a periphery of a lateral condyle of the proximal tibia and outlining a periphery of a medial condyle of the proximal tibia.

12. The computer program product (80) of any preceding claim, wherein the instructions are executable by one or more processors to utilize the pre-morbid bone model (PMF, PMT) to generate at least one of: a surgical plan for the morbid bone model (FBM, TBM) or a treatment plan for the morbid bone model (FBM, TBM).

13. The computer program product (80) of any preceding claim, wherein the instructions are executable by one or more processors to utilize the pre-morbid bone model (PMF, PMT) to generate a diagnosis for the morbid bone model (FBM, TBM).

14. A computer-implemented method (100, 200) for implementing the instructions of the computer program product (80) of any preceding claim.
